# EUROPEAN PATENT SPECIFICATION

(11) **EP 2 453 006 B1**
(45) Date of publication and mention of the grant of the patent: **07.10.2015**
(21) Application number: 10797237.4
(22) Date of filing: 28.05.2010
(51) Int. Cl.: C12N 1/21, C12N 15/53, C12P 13/08, C12N 15/52

(54) **METHOD FOR PRODUCING L-LYSINE USING CORYNEBACTERIUM SP. THAT HAS OBTAINED THE ACTIVITY OF GLYCERALDEHYDE-3-PHOSPHATE DEHYDROGENASE DERIVED FROM AN ALIEN SPECIES**
VERFAHREN ZUR HERSTELLUNG VON L-LYSIN MIT CORYNEBACTERIUM SP. MIT AUS EINER FREMDEN SPEZIES ABGELEITETER GLYCERALDEHYD-3-PHOSPHAT-DEHYDROGENASE-WIRKUNG
PROCÉDÉ DE PRODUCTION DE L-LYSINE PAR LE CORYNEBACTERIUM SP. À ACTIVITÉ ACQUISE DE GLYCERALDÉHYDE-3-PHOSPHATE DÉSHYDROGÉNASE PROVENANT D'UNE ESPÈCE ÉTRANGÈRE

(30) Priority: 08.07.2009 KR 20090062322
(43) Date of publication of application: 16.05.2012
(73) Proprietor: CJ CheilJedang Corporation, Seoul, 100-400 (KR)
(72) Inventor: RAH, So-yeon, Seoul 156-847 (KR); LIM, Sangjo, Incheon 402-040 (KR)
(74) Representative: Forrest, Graham Robert
(86) International application number: PCT/KR2010/003432
(87) International publication number: WO 2011/004962

(56) References cited:
- WO-A1-2006/071086
- KR-A- 20060 078 687
- ABDELGHANI IDDAR ET AL: 'Purification of recombinant non-phosphorylating NADP-dependent glyceraldehyde-3-phosphate dehydrogenase from Streptococcus pyogenes expressed in E. coli' MOLECULAR AND CELLULAR BIOCHEMISTRY vol. 247, no. 1-2, 2003, pages 195 - 203, XP008095145
- M. IKEDA ET AL: 'The Corynebacterium glutamicum genome: features and impacts on biotechnological processes' APPLIED MICROBIOLOGY AND BIOTECHNOLOGY vol. 62, no. 2-3, 2003, pages 99 - 109, XP001184751
- KOICHI TOYODA ET AL: 'Expression of the gapA gene encoding glyceraldehyde-3-phosphate dehydrogenase of Corynebacterium glutamicum is regulated by the global regulator SugR' APPLIED MICROBIOLOGY AND BIOTECHNOLOGY vol. 81, no. 2, 2008, pages 291 - 301, XP019654169
- SUNG OK HAN ET AL: 'Expression of Corynebacterium glutamicum glycolytic genes varies with carbon source and growth phase' MICROBIOLOGY vol. 153, no. 7, 2007, pages 2190 - 2202, XP008149906

<110> CJ CHEILJEDANG Corporation
<120> The Method of L-lysine production in corynebacteirum sp. by amplifying glyceraldehyde-3-phophate dehydrogenase gene derived from other
<130> OPA10051/PCT
<150> KR10-2009-0062322
   <151> 2009-07-08
<160> 14
<170> KopatentIn 1.71
<210> 1
   <211> 26
   <212> DNA
   <213> Artificial Sequence
<220>
   <223> Streptococcus mutans gapN_F1 primer
<400> 1
   gcgcatatga caaaacaata taaaaa 26
<210> 2
   <211> 27
   <212> DNA
   <213> Artificial Sequence
<220>
   <223> Streptococcus mutans gapN_R1 primer
<400> 2
   gcgtctagat tatttgatat caaatac 27
<210> 3
   <211> 24
   <212> DNA
   <213> Artificial Sequence
<220>
   <223> Streptococcus agalactiae gapN_F2 primer
<400> 3
   gcgcatatga caaaagaata tcaa 24
<210> 4
   <211> 27
   <212> DNA
   <213> Artificial Sequence
<220>
   <223> Streptococcus agalactiae gapN_R2 primer
<400> 4
   gcgtctagac tatttcatat caaaaac 27
<210> 5
   <211> 24
   <212> DNA
   <213> Artificial Sequence
<220>
   <223> Bacillus cereus gapN_F3 primer
<400> 5
   gcgcatatga caactagcaa tacg 24
<210> 6
   <211> 24
   <212> DNA
   <213> Artificial Sequence
<220>
   <223> Bacillus cereus gapN_R3 primer
<400> 6
   gcgtctagat taaactaagt ttaa 24
<210> 7
   <211> 24
   <212> DNA
   <213> Artificial Sequence
<220>
   <223> Corynebacterium glutamicum KFCC10881 F- gapA -SalI_P1 primer
<400> 7
   cacgtcgacg aatgtgtctg tatg 24
<210> 8
   <211> 24
   <212> DNA
   <213> Artificial Sequence
<220>
   <223> Corynebacterium glutamicum KFCC10881 R- gapA- XbaI_P2 primer
<400> 8
   tgatctagaa gatgatgacc ttct 24
<210> 9
   <211> 27
   <212> DNA
   <213> Artificial Sequence
<220>
   <223> Corynebacterium glutamicum KFCC10881 F- gapA -XbaI_P3 primer
<400> 9
   ccatctagag ctctggttct cccagag 27
<210> 10
   <211> 27
   <212> DNA
   <213> Artificial Sequence
<220>
   <223> Corynebacterium glutamicum KFCC10881 R- gapA -XbaI_P4 primer
<400> 10
   gcttctagag gtcttaacag ccatgcc 27
<210> 11
   <211> 1428
   <212> DNA
   <213> Streptococcus mutans
<400> 11
   ttgacaaaac aatataaaaa ttatgtcaat ggcgagtgga agctttcaga aaatgaaatt      60
   aaaatctacg aaccggccag tggagctgaa ttgggttcag ttccagcaat gagtactgaa     120
   gaagtagatt atgtttatgc ttcagccaag aaagctcaac cagcttggcg atcactttca     180
   tacatagaac gtgctgccta ccttcataag gtagcagata ttttgatgcg tgataaagaa     240
   aaaataggtg ctgttctttc caaagaggtt gctaaaggtt ataaatcagc agtcagcgaa     300
   gttgttcgta ctgcagaaat cattaattat gcagctgaag aaggccttcg tatggaaggt     360
   gaagtccttg aaggcggcag ttttgaagca gccagcaaga aaaaaattgc cgttgttcgt     420
   cgtgaaccag taggtcttgt attagctatt tcaccattta actaccctgt taacttggca     480
   ggttcgaaaa ttgcaccggc tcttattgcg ggaaatgtta ttgcttttaa accaccgacg     540
   caaggatcaa tctcagggct cttacttgct gaagcatttg ctgaagctgg acttcctgca     600
   ggtgtcttta ataccattac aggtcgtggt tctgaaattg gagactatat tgtagaacat     660
   caagccgtta actttatcaa tttcactggt tcaacaggaa ttggggaacg tattggcaaa     720
   atggctggta tgcgtccgat tatgcttgaa ctcggtggaa aagattcagc catcgttctt     780
   gaagatgcag accttgaatt gactgctaaa aatattattg caggtgcttt tggttattca     840
   ggtcaacgct gtacagcagt taaacgtgtt cttgtgatgg aaagtgttgc tgatgaactg     900
   gtcgaaaaaa tccgtgaaaa agttcttgca ttaacaattg gtaatccaga agacgatgca     960
   gatattacac cgttgattga tacaaaatca gctgattatg tagaaggtct tattaatgat    1020
   gccaatgata aaggagccgc tgcccttact gaaatcaaac gtgaaggtaa tcttatctgt    1080
   ccaatcctct ttgataaggt aacgacagat atgcgtcttg cttgggaaga accatttggt    1140
   cctgttcttc cgatcattcg tgtgacatct gtagaagaag ccattgaaat ttctaacaaa    1200
   tcggaatatg gacttcaggc ttctatcttt acaaatgatt tcccacgcgc ttttggtatt    1260
   gctgagcagc ttgaagttgg tacagttcat atcaataata agacacagcg cggtacggac    1320
   aacttcccat tcttaggggc taaaaaatca ggtgcaggta ttcaaggggt aaaatattct    1380
   attgaagcta tgacaactgt taaatccgtc gtatttgata tcaaataa                 1428
<210> 12
   <211> 1428
   <212> DNA
   <213> Streptococcus agalactiae
<400> 12
   ttgacaaaag aatatcaaaa ttatgtcaat ggcgaatgga aatcatctgt taatcagatt      60
   gagattttgt caccaattga tgattcttca ttgggattcg tgccagcgat gactcgagaa     120
   gaagttgatc atgctatgaa agcgggtcgt gaggctttac cagcctgggc tgctttgaca     180
   gtatatgaac gtgcacaata ccttcataaa gccgcagaca ttattgaacg tgataaagaa     240
   gaaattgcta ctgttttagc aaaagaaatt tctaaagctt acaatgcttc agtaactgag     300
   gttgtaagga cagctgatct tattcgttat gcagcagaag aaggaattcg tttatcaact     360
   tcagctgacg aaggtggaaa aatggatgct tcaacaggtc ataagttggc tgttattcgt     420
   cgtcaaccag taggtatcgt tttagcaatc gcaccttata attaccctgt taacctctca     480
   ggatcaaaaa ttgcgccagc tctaattggt ggaaacgttg tgatgtttaa accaccaaca     540
   caaggttcag tctcaggact tgttttagca aaagcttttg cagaagcagg tcttccagca     600
   ggtgtcttta atactattac aggacgcggt tctgagattg gagattacat tgttgagcat     660
   gaagaagtta attttattaa ctttacagga tcaacgccag ttggaaaacg tattggtaag     720
   ttagcaggaa tgcgtccaat tatgcttgag ttgggtggta aggatgcagg tgtcgtctta     780
   gctgatgctg accttgacaa cgctgctaaa caaatcgttg caggtgctta tgattactcc     840
   ggacaacgct gtacggcaat taagcgtgta cttgtcgttg aagaagttgc agatgaattg     900
   gcagaaaaaa tatctgaaaa tgtagcaaaa ttatcagtag gtgatccatt tgataatgca     960
   acggtgacac cggttattga tgataattca gccgacttta ttgaaagctt agtagtagat    1020
   gcacgtcaaa aaggtgcgaa agaattgaat gaatttaaac gtgatggtcg tctattaact    1080
   ccaggattgt ttgatcatgt tactttagat atgaaactag cttgggaaga gccttttgga    1140
   ccaattctcc caattattcg tgtcaaggat gcagaagaag ctgttgctat tgccaacaaa    1200
   tctgattttg gattacaatc atcagtcttt acacgtgatt tccaaaaagc atttgatata    1260
   gcaaataaac ttgaagttgg tacagttcac attaacaata agactggacg tggtcctgat    1320
   aatttcccat tcttaggact caaaggatct ggtgcaggtg ttcaaggtat cagatattca    1380
   attgaagcaa tgacaaatgt aaaatcgata gtttttgata tgaaatag                 1428
<210> 13
   <211> 1440
   <212> DNA
   <213> Bacillus cereus
<400> 13
<210> 14
   <211> 1005
   <212> DNA
   <213> Corynebacterium sp.
<400> 14
   atgaccattc gtgttggtat taacggattt ggccgtatcg gacgtaactt cttccgcgca      60
   gttctggagc gcagcgacga tctcgaggta gttgcagtca acgacctcac cgacaacaag     120
   accctttcca cccttctcaa gttcgactcc atcatgggcc gccttggcca ggaagttgaa    180
   tacgacgatg actccatcac cgttggtggc aagcgcatcg ctgtttacgc agagcgcgat     240
   ccaaagaacc tggactgggc tgcacacaac gttgacatcg tgatcgagtc caccggcttc     300
   ttcaccgatg caaacgcggc taaggctcac atcgaagcag gtgccaagaa ggtcatcatc     360
   tccgcaccag caagcaacga agacgcaacc ttcgtttacg gtgtgaacca cgagtcctac     420
   gatcctgaga accacaacgt gatctccggc gcatcttgca ccaccaactg cctcgcacca     480
   atggcaaagg tcctaaacga caagttcggc atcgagaacg gcctcatgac caccgttcac     540
   gcatacactg gcgaccagcg cctgcacgat gcacctcacc gcgacctgcg tcgtgcacgt     600
   gcagcagcag tcaacatcgt tcctacctcc accggtgcag ctaaggctgt tgctctggtt     660
   ctcccagagc tcaagggcaa gcttgacggc tacgcacttc gcgttccagt tatcaccggt     720
   tccgcaaccg acctgacctt caacaccaag tctgaggtca ccgttgagtc catcaacgct     780
   gcaatcaagg aagctgcagt cggcgagttc ggcgagaccc tggcttactc cgaagagcca     840
   ctggtttcca ccgacatcgt ccacgattcc cacggctcca tcttcgacgc tggcctgacc     900
   aaggtctccg gcaacaccgt caaggttgtt tcctggtacg acaacgagtg gggctacacc     960
   tgccagctcc tgcgtctgac cgagctcgta gcttccaagc tctaa                    1005

## Description

### BACKGROUND

### 1. Field

The present disclosure relates to a *Corynebacterium* sp. strain having an activity of NADP-dependent glyceraldehyde-3-phosphate dehydrogenase and an improved productivity of L-lysine, and a method for producing L-lysine using the same.

### 2. Description of the Related Art

Traditionally, Coryneform bacteria are industrial microorganisms which are most widely used for the production of a variety of chemical materials useful in the animal feed, medicine and food industries, including amino acids, such as L-lysine, L-threonine, L-arginine, L-threonine and glutamic acid, and nucleic acid-related materials. These microorganisms are Gram-positive and require biotin for their growth. Representative examples of coryneform bacteria are the genus *Corynebacterium* including *Corynebacterium glutamicum,* the genus *Brevibacterium* including *Brevibacterium* including *Brevibacterium flavum*, the species *Arthrobacter*, the species *Microbacterium*, etc.

L-lysine is an L-amino acids, which is commercially used as a feed additive in animal nutrition due to its ability to help the body absorb other amino acids thereby improving the quality of feedstuff. For the human body, L-lysine is used as an ingredient of an injection solution, and also finds applications in the pharmaceutical field. Therefore, the industrial production of lysine is an economically important industrial process.

To improve the production yield of lysine, the enzyme activity on the lysine biosynthetic pathway has been typically enhanced by amplifying genes on the lysine biosynthetic pathway or by modifying promoter of the genes. In addition, an exogenous gene derived from other bacteria can be introduced, and for example, introduction of a gene coding for citrate synthase derived from *Escherichia coli* is described in Japanese Patent Publication No. Hei 7-121228.

Meanwhile, in vivo central carbon metabolic pathway can be modified in order to improve lysine productivity. involved in the central carbon metabolic pathway. In *Corynebacteria*, it exists in a form of gapA which converts glyceraldehyde-3-phosphate into glycerate-1,3-bisphosphate using NAD as a coenzyme, in which glycerate-1,3-bisphosphate is converted into 3-phosphoglycerate by the pgk gene encoding enzyme. On the contrary, in *streptococcus* and *Bacillus,* it exists as a non-phosphorylating NADP-dependent GAPDH (non-phosphorylating NADP-dependent glyceraldehyde-3-phosphate dehydrogenase, hereinbelow, referred to as gapN) which converts glyceraldehyde-3-phosphate into 3-phosphoglycerate using NADP as a coenzyme, and produces NADPH. This process is a one-step process of playing an important role in glycolysis, and is affected by a NADPH/NADP ratio (Iddar, A et al., Protein Expr Purif. 25(3):519-26(2002)).

WO 2006/071086 describes *Escherichia* species microorganism and Corynebacterium species microorganism that are transformed with a foreign NADP dependent glyceraldehydes-3-phosphate dehydrogenase gene and have the ability to produce L-lysine and a method of producing L-lysine using the microorganisms.

It was reported that the enzyme gapN does not inherently exist in *Corynebacterium*. Also, there is no report of using the gapN gene in the culture of *Corynebacterium.* However, there are prior patents of increasing the amino acid productivity by introduction of the gapN gene. For example, Korean Patent Application No. 10-2004-0116999 and US Patent No. 11/722,820 describe that the gapN is expressed in *E.coli* to increase lysine productivity, but there is no report of applying it to *Corynebacterium.* Therefore, in order to achieve the effect of gapN gene, the *Clostridium acetobutyricum*-derived gapN was intended to be expressed in the lysine-producing *Corynebacterium* strain, but a satisfactory result could not be obtained. Thus, the present authors have explored three types of gapN genes present in foreign bacteria through paper search (Abdelaziz Soukri et al. INTERNATIONAL MICROBIOLOGY (2005) 8:251-258)

The present authors found that the gapN is expressed in order to use NADP, instead of NAD, as a coenzyme, and the resulting increase in NADPH levels is used as the source of reducing power for lysine biosynthesis, and thus the L-lysine productivity of *Corynebacterium* sp. strain can be enhanced, thereby completing the present disclosure.

### SUMMARY

An object is to provide a *Corynebacterium* sp. strain having an activity of NADP-dependent glyceraldehyde-3-phosphate dehydrogenase and an improved productivity of L-lysine, and a method for producing L-lysine using the same.

### BRIEF DESCRIPTION OF THE DRAWINGS

FIG. 1 shows a cleavage map of pECCG122-Pcj7-gapN, which is a expression plasmid for glyceraldehyde-3-phosphate dehydrogenase (gapN) of *Streptococcus mutans, Streptococcus agalactiae*, or *Bacillus cereus;* and
FIG. 2 shows a cleavage map of a PDZ-gapA chromosomal insertion vector for disruption of the gapA gene.

### DETAILED DESCRIPTION OF THE PREFERRED EMBODIMENTS

In one embodiment, the present disclosure provides a *Corynebacterium* sp. strain having an activity of NADP-dependent glyceraldehyde-3-phosphate dehydrogenase and an improved productivity of L-lysine.

The *Corynebacterium* sp. strain having an improved productivity of L-lysine of the present disclosure may be obtained by inactivating the endogenous glyceraldehyde-3-phosphate dehydrogenase-encoding gene (gapA) and introducing the exogenous NADP-dependent glyceraldehyde-3-phosphate dehydrogenase-encoding gene (gapN).

In the present disclosure, the exogenous NADP-dependent glyceraldehyde-3-phosphate dehydrogenase-encoding gene includes any nucleotide encoding a polypeptide, as long as the polypeptide has an activity of converting into 3- phosphoglycerate by using glyceraldehyde-3-phosphate as a substrate and NADP as a coenzyme.

Examples of exogenous NADP-dependent glyceraldehyde-3-phosphate dehydrogenase-encoding gene include NADP-dependent glyceraldehyde-3-phosphate dehydrogenase-encoding genes that are derived from animals, plants and bacteria, preferably, NADP-dependent glyceraldehyde-3-phosphate dehydrogenase-encoding genes that are derived from bacteria, and most preferably, an NADP-dependent glyceraldehyde-3-phosphate dehydrogenase-encoding gene that is originated from *Streptococcus mutans*, *Streptococcus agalactiae*, or *Bacillus* cereus. More specifically, the genes of *Streptococcus mutans, Streptococcus agalactiae,* and *Bacillus* cereus may have the nucleotide sequences of SEQ ID NOS. 11 (GenBank accession No. NC_004350), 12 (GenBank accession No. NC_004116), and 13 (GenBank accession No. NC_004722.1), respectively.

The exogenous NADP-dependent glyceraldehyde-3-phosphate dehydrogenase-encoding gene may be introduced into a expressing recombinant vector; and introducing the recombinant vector into a coryneform bacterium having an inactivated endogenous glyceraldehyde-3-phosphate dehydrogenase gene so as to produce a transformed coryneform bacterium.

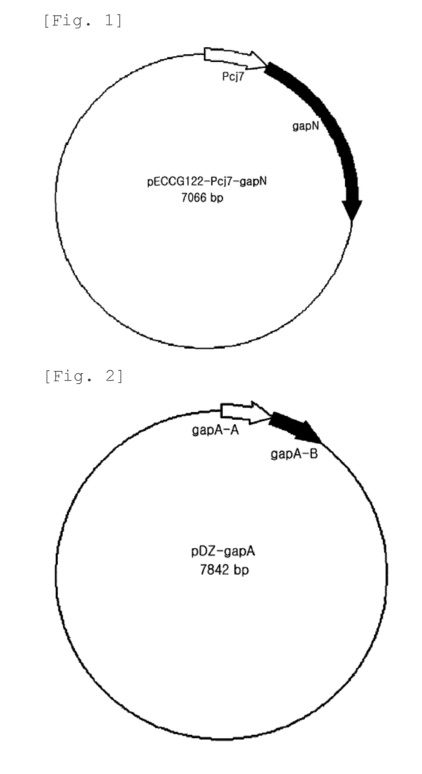

The NADP-dependent glyceraldehyde-3-phosphate dehydrogenase-expressing recombinant vector may be prepared by a common method, for example, ligating the sequence of the exogenous NADP-dependent glyceraldehyde-3-phosphate dehydrogenase gene to a suitable vector using a restriction enzyme. In the present disclosure, the vector may be a vector having the cleavage map of FIG. 1. In the specific embodiment of the present disclosure, the vectors for the introduction of the genes of *Streptococcus mutants, Streptococcus agalactiae*, and *Bacillus* cereus are pECCG122-Prj7-gapN1, pECCG122-Pcj7-gapN2, and pECCG122-Pcj7-gapN3, respectively.

The endogenous glyceraldehyde-3-phosphate dehydrogenase-encoding gene refers to a gene encoding the enzyme that has an activity of converting into glycerate-1,3-bisphosphate by using glyceraldehyde-3-phosphate as a substrate and NAD as a coenzyme in the *Corynebacterium* sp. strain, and preferably refers to a gene having the nucleotide sequence of SEQ ID NO. 14 (NCBI Accession No. NC_003450, NCgl1526)

Further, in order to inactivate the endogenous glyceraldehyde-3-phosphate dehydrogenase-encoding gene, genetic manipulation of deletion, substitution or insertion of the native gapA gene of the *Corynebacterium* sp. strain may be performed. Preferably, a coryneform bacterium may be transformed with a recombinant vector including a part of the gapA gene, and for example, a strain having the inactivated gapA activity may be prepared by introduction of a vector having the cleavage map of FIG. 2. In the specific embodiment of the present disclosure, a pDZ-gapA recombinant vector having two fragments of a part of the endogenous glyceraldehyde-3-phosphate dehydrogenase-encoding gene consecutively cloned was used as a vector for chromosomal insertion. Preferably, a coryneform bacterium to be transformed with this vector for chromosomal insertion is, but not limited to, *Corynebacterium glutamicum* KFCC-10881. According to the specific embodiment of the present disclosure, the coryneform bacterium having the disruption of the endogenous glyceraldehyde-3-phosphate dehydrogenase-encoding gene may be *Corynebacterium glutamicum* CA01-0096 (Accession No. KCCM 11012P).

As used herein, the term "transformation" means that DNA is introduced into a host wherein the DNA remains as an extrachromosomal element or integrated into the chromosome and invention, the coryneform bacterium having the disruption of the endogenous glyceraldehyde-3-phosphate dehydrogenase-encoding gene may be *Corynebacterium glutamicum* CA01-0096 (Accession No. KCCM 11012P).

As used herein, the term "transformation" means that DNA is introduced into a host wherein the DNA remains as an extrachromosomal element or integrated into the chromosome and is made to be replicable. As used herein, the term "transfection" means that an expression vector having an arbitrary coding sequence which may be expressed or not is received by a host cell. As used herein, the terms "transfected host" and "transformed host" mean a cell into which DNA is introduced. The cell is called a "host cell", and it may be a prokaryotic or eukaryotic cell. The typical prokaryotic cell includes a variety of strains such as *E. coli.* The term "vector" refers to a DNA product having a DNA sequence operably linked to a regulatory sequence that can express DNA in a suitable host. Examples of the regulatory sequence include a promoter for transcription, an arbitrary operator sequence for regulating transcription, a sequence encoding an appropriate mRNA ribosome binding site, and sequences for regulating the termination of transcription and translation. Examples of the vector may include plasmids, phage particles, or simply potential genomic inserts. Once transformed into a suitable host, the vector may replicate and function independently of the host genome, or may, in some instances, integrate into the genome itself. As used herein, "plasmid" and "vector" are sometimes used interchangeably as the plasmid is the most commonly used form of vector at present. The term "regulatory sequence" refers to a DNA sequence necessary for the expression of the operably linked coding sequence in a particular host organism. For example, regulatory sequences necessary for expression in prokaryotes include a promoter, optionally an operator sequence, and a ribosome binding site. Eukaryotes are known to use a promoter, a polyadenylation signal, and an enhancer.

When a nucleic acid is disposed in a functional relationship with other nucleic acid sequence, it is "operably linked" to the nucleic acid sequence. It means that genes are linked in such a manner as to enable expression of the gene when the regulatory sequence(s) couples with an appropriate molecule (e.g., a transcription-activating protein). For example, DNA of a pre-sequence or a secretory leader is operably linked to DNA of polypeptide when it is expressed as a pre-protein that participates in secretion of polypeptide; a promoter or enhancer is operably linked to a coding sequence when it affects transcription of the coding sequence; a ribosome binding site is operably restriction enzyme site, a synthetic oligonucleotide adaptor or linker may be used according to an ordinary method.

As used herein, the term "vector" refers to a recombinant carrier into which heterologous DNA fragments are inserted, in which the DNA fragment is generally a double-strand DNA fragment. Here, heterologous DNA means hetero-type DNA that is not naturally found in a host cell. Once the expression vector is incorporated with a host cell, it can be replicated irrespective of host genomic DNA to generate several copies and their inserted (heterologous) DNAs.

As is known to one skilled in the art, in order to raise an expression level of a transfected gene in a host cell, the corresponding gene must be operably linked to an expression control sequence that performs transcription and translation functions in a selected expression host. Preferably, the expression control sequence and the gene are included in a single expression vector comprising both a bacterial selectable marker and a replication origin. When an expression host is a eukaryotic cell, the expression vector must further include an expression marker useful in the eukaryotic expression host.

The vector useful for the preparation of the recombinant vector of the present disclosure may be derived from vectors autonomically replicable in the coryneform bacteria, and for example, a phage vector or a plasmid vector. Examples of the phage vector or the cosmid vector include pWE15, M13, λEMBL3, λEMBL4, λFIXII, λDASHII, λZAPII, λgt10, λgt11, Charon4A, and Charon21A, and examples of the plasmid vector include a pBR plasmid, a pUC plasmid, a pBluescriptII plasmid, a pGEM plasmid, a pTZ plasmid, a pET plasmid, a pMal plasmid, a pQE plasmid, etc.

Further, the *Corynebacterium* sp. used in the present disclosure includes any *Corynebacterium* sp., as long as it has L-lysine productivity. Examples of the *Corynebacterium* sp. include *Corynebacterium glutamicum, Corynebacterium thermoaminogenes, Brevibacterium flavum,* and *Brevibacterium fermentum*.

The *Corynebacterium* sp. of the present disclosure includes a mutant strain having enhanced L-lysine productivity as well as a wild-type strain. Examples thereof include strains that nutritionally require methionine or are resistant to threonine analogues (AHV: α-amino-β-hydroxy valeric acid), lysine analogues (AEC: S-(2-aminoethyl)-L-cysteine), isoleucine analogues (α-aminobutyric acid), methionine analogues (ethionine), etc. In addition, in order to improve the L-lysine productivity, the strain may be a mutant having an improved L-lysine productivity that is prepared by increasing the copy number of the introduced gene or manipulating a gene regulatory sequence of the gene. Preferably, it may include *Corynebacterium glutamicum* ATCC13032, *Corynebacterium thermoaminogenes* FERM BP-1539, *Brevibacterium flavum* ATCC 14067, *Brevibacterium lactofermentum* ATCC 13869, and an L-amino acid-producing mutant or strain prepared therefrom, for example, *Corynebacterium glutamicum* KFCC11001, and most preferably, *Corynebacterium glutamicum* KFCC10881, but is not limited thereto.

The specific embodiment of the present disclosure provides *Corynebacterium* sp. strains of *Corynebacterium glutamicum* CA01-0565 (Accession No. KCCM 11013P), *Corynebacterium glutamicum* CA01-0566 (Accession No. KCCM 11014P), and *Corynebacterium glutamicum* CA01-0567 (Accession No. KCCM 11015P), which are prepared by introducing the gene of *Streptococcus mutans,* the gene of *Streptococcus agalactiae,* and the gene of *Bacillus* cereus into the Corynebacterium glutamicum KFCC10881 having the inactivated endogenous glyceraldehyde-3-phosphate dehydrogenase-encoding gene, respectively. These strains were deposited in KCCM (Korean Culture Center of Microorganisms, located at 361-221 Yurim Bild., Hongje 1-dong, Seodaemun-gu, Seoul, Korea) on July 2, 2009.

The improvement in L-lysine productivity is attributed to the increased reducing power by activation of NADP-dependent glyceraldehyde-3-phosphate dehydrogenase. Instead of the endogenous glyceraldehyde-3-phosphate dehydrogenase, an exogenous NADP-dependent glyceraldehyde-3-phosphate dehydrogenase is introduced into the L-lysine-producing *Corynebacterium* strain, so that NADP-dependent glyceraldehyde-3-phosphate dehydrogenase is activated to make the strain to utilize NADP instead of NAD as a coenzyme, and thus the resulting increase in NADPH levels can be used as the source of reducing power for L-lysine biosynthesis.

Another embodiment of the present disclosure provides a method for producing L-lysine, comprising the steps of culturing the *Corynebacterium* sp. strain having an activity of NADP-dependent glyceraldehyde-3-phosphate dehydrogenase and an improved productivity of L-lysine, and recovering lysine from the cultured cells or the culture broth.

The *Corynebacterium* sp. strain used according to the present disclosure may be cultured by continuous or batch type method such as batch, fed-batch and repeated fed-batch cultures. A summary of known cultivation methods is described in the textbook [Chmiel, (Bioprozesstechnik 1. Einfuhrung in die Bioverfahrenstechnik (Gustav Fischer Verlag, Stuttgart, 1991); and Storhas (Bioreaktoren und periphere Einrichtungen (Vieweg Verlag, Braunschweig/Wiesbaden, 1994)]. of NADP-dependent glyceraldehyde-3-phosphate dehydrogenase and an improved productivity of L-lysine, and recovering lysine from the cultured cells or the culture broth.

The *Corynebacterium* sp. strain used according to the present invention may be cultured by continuous or batch type method such as batch, fed-batch and repeated fed-batch cultures. A summary of known cultivation methods is described in the textbook [Chmiel, (Bioprozesstechnik 1. Einfuhrung in die Bioverfahrenstechnik (Gustav Fischer Verlag, Stuttgart, 1991); and Storhas (Bioreaktoren und periphere Einrichtungen (Vieweg Verlag, Braunschweig/Wiesbaden, 1994)].

The culture medium to be used must meet the requirements of the particular strains in a suitable manner. Descriptions of culture media for *Corynebacterium* sp. strain are to be found in the handbook ["Manual of Methods for General Bacteriology" by the American Society for Bacteriology (Washington D.C., USA, 1981)]. The useful carbon source may include sugars and carbohydrates such as glucose, saccharose, lactose, fructose, maltose, starch and cellulose, oils and fats such as soybean oil, sunflower oil, groundnut oil and coconut oil, fatty acids such as palmitic acid, stearic acid and linoleic acid, alcohols such as glycerol and ethanol, and organic acids such as acetic acid. vitamins, may be used in addition to the above-mentioned substances. Suitable precursors may be also added to the culture medium. The mentioned substances may be added to the culture by continuous or batch type in a suitable manner during the cultivation.

In order to control the pH value of the culture, basic compounds such as sodium hydroxide, potassium hydroxide, and ammonia or acid compounds such as phosphoric acid or sulfuric acid, are expediently used. In order to control the development of foam, an anti-foaming agent such as fatty acid polyglycol esters may be used. In order to maintain aerobic conditions, oxygen or oxygen-containing gas such as air is introduced into the culture. The temperature of the culture is normally from 20°C to 45°C, and preferably from 25°C to 40°C. The culture is continued until the maximum amount of the desired L-amino acids has formed. That aim is normally achieved within a period of from 10 hours to 160 hours.

The analysis of L-amino acids may be carried out by anion-exchange chromatography with subsequent ninhydrin derivatization (Spackman et al. (Analytical Chemistry, 30, (1958), 1190).

The present disclosure will be described in more detail with reference to the following Examples. However, the disclosure is not intended to be limited by these Examples.

### Example 1. Exploration and cloning of gapN gene of Streptococcus mutans

The sequence of the *Streptococcus*-derived gapN gene has been clearly revealed. The gapN gene information of *Streptococcus mutans* (accession No. NC_004350) was acquired from NIH GenBank. Based on the reported sequence, a pair of primers described in the following Table 1 was synthesized and 1428 base pairs of the gapN gene were amplified by Polymerization Chain Reaction (PCR) [Sambrook et al, Molecular Cloning, a Laboratory Manual (1989), Cold Spring Harbor Laboratories](PCR conditions: Denaturing=94°C, 30 sec/Annealing=50°C, 30 sec/Polymerization=72°C, 1 min 30 sec, 30 cycles) using a chromosomal DNA of the *Streptococcus mutans* ATCC25175 strain as a template, and cloned into an *E. coli* plasmid pCR2.1 using a TOPO Cloning Kit (Im-itrogen) so as to obtain a pCR-gapN1 plasmid.

**[Table 1] Primer**

| Primer | sequence | SEQ ID NO. |
|---|---|---|
| gapN_F1 | 5'-GCG CAT ATG ACA AAA CAA TAT AA AAA-3' | 1 |

Polymerization Chain Reaction (PCR) [Sambrook et al, Molecular Cloning, a Laboratory Manual (1989), Cold Spring Harbor Laboratories](PCR conditions: Denaturing=94°C, 30 sec/Annealing=50°C, 30 sec/Polymerization=72°C, 1 min 30 sec, 30 cycles) using a chromosomal DNA of the *Streptococcus mutans* ATCC25175 strain as a template, and cloned into an *E. coli* plasmid pCR2.1 using a TOPO Cloning Kit (Invitrogen) so as to obtain a pCR-gapN1 plasmid.

**[Table 1] Primer**

| Primer | sequence | SEQ ID NO. |
|---|---|---|
| gapN_F1 | 5'-GCG CAT ATG ACA AAA CAA TAT AA AAA-3' | 1 |
| gapN_R1 | | 2 |

### Example 2. Construction of gapN expression vector

The gapN gene-containing pCR-gapN1 vector obtained in Example 1 was cleaved using restriction enzymes Nde-I and Xba-I, and the gapN-encoding gene fragment was only separated, and an expression promoter Pcj7 (reference; Patent Publication No. 2006-0068505) was ligated with a shuttle vector pECCG122 of *E. coli* and *Corynebacterium* (reference; Patent Publication No. 1992-0000933), so as to construct pECCG122-Pcj7-gapN1 (FIG. 1).

### Example 3. Exploration and cloning of gapN gene of Streptococcus agalactie

The sequence of the *Streptococcus*-derived gapN gene has been clearly revealed. The gapN gene information of *Streptococcus agalactie* (accession No. NC_004116) was acquired from NIH GenBank. Based on the reported sequence, a pair of primers described in the following Table 2 was synthesized and 1428 base pairs of the gapN gene were amplified by Polymerization Chain Reaction (PCR) [Sambrook et al, Molecular Cloning, a Laboratory Manual (1989), Cold Spring Harbor Laboratories](PCR conditions: Denaturing=94°C, 30 sec/Annealing=50°C, 30 sec/Polymerization=72°C, 1 min 30 sec, 30 cycles) using a chromosomal DNA of the *Streptococcus agalactie* ATCC BAA-611 strain as a template, and cloned into an *E. coli* plasmid pCR2.1 using a TOPO Cloning Kit (Invitrogen) so as to obtain a pCR-gapN2 plasmid.

**[Table 2] Primer**

| Primer | sequence | SEQ ID NO. |
|---|---|---|
| gapN_F2 | 5'-GCG CAT ATG ACA AAA GAA TAT CAA-3' | 3 |
| gapN_R2 | | 4 |

### Example 4. Construction of gapN expression vector

The gapN gene-containing pCR-gapN 2 vector obtained in Example 3 was cleaved using restriction enzymes Nde-I and Xba-I, and the gapn-encoding gene fragment was only separated, and an expression promoter Pcj7 (reference; Patent Publication No. 2006-0068505) was ligated with a shuttle vector pECCG122 of *E.coli* and *Corynebacterium* (reference; Patent Publication No. 1992-0000933), so as to construct pECCG122-Pcj7-gapN2 (FIG. 1).

### Example 5. Exploration and cloning of gapN gene of Bacillus cereus

The sequence of the *Bacillus*-derived gapN gene has been clearly revealed. The gapN gene information of *Bacillus cereus* (accession No. NC_004722.1) was acquired from NIH GenBank. Based on the reported sequence, a pair of primers described in the following Table 3 was synthesized and 1428 base pairs of the gapN gene were amplified by Polymerization Chain Reaction (PCR) [Sambrook et al, Molecular Cloning, a Laboratory Manual (1989), Cold Spring Harbor Laboratories](PCR conditions: Denaturing=94°C, 30 sec/Annealing=50°C, 30 sec/Polymerization=72°C, 1 min 30 sec, 30 cycles) using a chromosomal DNA of the *Bacillus cereus* ATCC 14579 strain as a template, and cloned into an *E. coli* plasmid pCR2.1 using a TOPO Cloning Kit (Invitrogen) so as to obtain a pCR-gapN3 plasmid.

**[Table 3] Primer**

| Primer | sequence | SEQ ID NO. |
|---|---|---|
| gapN_F3 | 5'-GCG CAT ATG ACA ACT AGC AAT ACG-3' | 5 |
| gapN_R3 | 5'-GCG TCT AGA TTA AAC TAA GTT TAA-3' | 6 |

### Example 6. Construction of gapN expression vector

The gapN gene-containing pCR-gapN3 vector obtained in Example 5 was cleaved using restriction enzymes Nde-I and Xba-I, and the gapN-encoding gene fragment was only separated, and an expression promoter Pcj7 was ligated with a shuttle vector pECCG122 of *E. coli* and *Corynebacterium,* so as to construct pECCG122-Pcj7-gapN3 (FIG. 1).

### Example 7. Preparation of lysine-producing Corynebacterium glutamicum (KFCC-10881) by artificial mutation

*Corynebacterium glutamicum* (KFCC-10881) prepared by artificial mutation, which is resistant to S-(2-aminoethyl) cysteine (hereinafter referred to as "AEC") and is homoserine-leaky, was used as a strain into which a plurality of genes responsible for lysine biosynthetic pathway were to be inserted.

The mutant strain KFCC-10881 was prepared from the wild-type *Corynebacterium glutamicum* (ATCC13032) as a mother strain. The mother strain of 10^{7~}10⁸ cells/ml was treated with the mutagen N-methyl-N'-nitro-N-nitrosoguanidine (hereinafter referred to as "NTG") in a final concentration of 500 µg/ml at 30°C for 30 min, followed by the selection of colonies grown on a complex plate containing 5 g/l of AEC. After the primary mutant strain was analyzed for AEC resistance and lysine productivity, it was led to secondary mutation with NTG. A plurality of the colonies thus formed were tooth-picked into minimal media, which were or were not supplemented with homoserine (100 mg/L), so as to separate homoserine auxotrophs (secondary mutants), which cannot grow in a minimal medium lacking homoserine. The homoserine auxotroph was allowed to undergo tertiary mutation so as to create a homoserine-leaky strain which was identified by incubation in a minimal medium containing 10 mg/L of homoserine. The strain grown in the medium was examined for lysine productivity (Table 4). The resulting lysine-producing strain, which is AEC resistant and homoserine-leaky, was deposited with the Korean Federation of Culture Collection under the accession number of KFCC-10881.

**[Table 4] Lysine productivity of KFCC-10881**

| Strain | Lysine (g/l) | | |
|---|---|---|---|
| | Batch 1 | Batch 2 | Batch 3 |
| Wild-type (ATCC13032) | 0 | 0 | 0 |
| KFCC-10881 | 45 | 43 | 42.5 |

### Minimal Medium (pH 7.0)

Glucose 10 g, (NH₄)₂SO₄ 5 g, urea 2 g, KH₂PO₄ 1 g, K₂HPO₄ 2 g, MgSO₄ 7H₂O 0.4 g, biotin 200 µg, thiamine HCl 3000 µg, Calcium Pantothenate 1000 µg, nicotinamide 5000 µg, NaCl 0.5 g (per 1 liter of distilled water)

### Seed Medium (pH 7.0)

Glucose 20 g, Peptone 10 g, Yeast Extract 10 g, urea 5 g, KH₂PO₄ 4 g, K₂HPO₄ 8 g, MgSO₄ 7 H₂O 0.5 g, biotin 100 µg, thiamine HCl 1000 µg (per 1 liter of process water)

### Production Medium (pH 7.0)

Glucose 100 g, (NH₄)₂SO₄ 40 g, Soy Protein 2.5 g, Corn Steep Solids 5 g, urea 3 g, KH₂PO₄ 1 g, MgSO₄ 7H₂O 0.5 g, biotin 100 µg, thiamine HCl 1000 µg, CaCO₃ 30 g (per 1 liter of distilled water)

### Example 8. Cloning of lysine-producing Corynebacterium glutamicum KFCC-10881-derived gapA gene, Construction of recombinant vector (pDZ-gapA), and Preparation of gapA-disrupted strain

In this Example, the gapA gene of lysine biosynthetic pathway was acquired by PCR using a chromosomal DNA of the lysine-producing *Corynebacterium glutamicum* KFCC10881 prepared in Example 7 as a template. Based on the NIH GenBank, sequence information of the gapN gene (NCBI accession No. NC_003450, NCg11526) was acquired, and two pairs of primers (Table 5, SEQ ID NOS. 7 to 10) were synthesized.

PCR was performed using the chromosomal DNA of *Corynebacterium glutamicum* KFCC10881 as a template and a set of oligonucleotide primers of SEQ ID NOS. 7 and 10 in the presence of PfuUltra™ High-Fidelity DNA Polymerase (Stratagene), with 30 cycles of denaturing at 96°C for 30 sec; annealing at 53°C for 30 sec; and polymerization at 72°C for 30 sec. The PCR products thus obtained were found to be two kinds of gapA gene fragment (gapA-A, gapA-B) of 600 bp. The gapA-A was amplified using the primers of SEQ ID NOS. 7 and 8, and the gapA-B was amplified using the primers of SEQ ID NOS. 9 and 10. The amplified products were cloned into an *E. coli* vector pCR2.1 using a TOPO Cloning Kit (Invitrogen), so as to obtain pCR-gapA-A and pCR-gapA-B vectors.

**[Table 5] Primer**

| Primer | sequence | SEQ ID NO. |
|---|---|---|
| F- gapA -SalI_P1 | CAC GTC GAC GAA TGT GTC TGT ATG | 7 |
| R- gapA- XbaI_P2 | TGA TCT AGA AGA TGA TGA CCT TCT | 8 |
| F- gapA -XbaI_P3 | CCA TCT AGA GCT CTG GTT CTC CCA GAG | 9 |
| R- gapA -XbaI_P4 | GCT TCT AGA GGT CTT AAC AGC CAT GCC | 10 |

These pCR vectors were treated respectively with the restriction enzymes included in each end of gapA-A and gapA-B (gapA-A: SalI, XbaI, gapA-B: XbaI) to separate the gapA genes from the pCR vectors. Next, these fragments were cloned through 3-piece ligation into a pDZ vector (reference: Patent Publication No. 10-2008-0025355) treated with restriction enzymes SalI and XbaI, so as to produce a pDZ-gapA recombinant vector, in which two copies of gapA were consecutively cloned. FIG. 2 shows a pDZ-gapA vector for chromosomal insertion of *Corynebacterium*.

The constructed pDZ-gapA vector was transformed into the lysine-producing *Corynebacterium glutamicum* KFCC-10881 prepared in Example 7, followed by insertion of a vector having 300 base pairs deletion within the gapA gene into the gapA gene on the chromosome through secondary crossover, so as to produce a lysine-producing *Corynebacterium glutamicum* CA01-0096 having the inactivated gapA gene (Accession No. KCCM 11012P). PCR was performed using the primers of SEQ ID NOS. 7 and 10 to confirm that the disrupted gapA gene has 300 base pairs deletion of the wild-type gapA gene.

### Example 9. Induction of gapN gene expression in CA01-0096

The pECCG122-Pcj7-gapN1~3 vectors obtained in Examples 2, 4, and 6 were introduced into CA01-0096. The gapA-disrupted strain is not known to grow in a minimal medium where *Corynebacterium glutamicum* usually grows (reference paper; Hideaki Yukawa et al., J Mol Microbiol Biotechnol 2004; 8:91-103).

The pECCG122-Pcj7-gapN1~3 vectors were transformed into CA01-0096 by an electrical pulse method so as to prepare transformed strains (Strain No. CA01-0565, CA01-0566, CA01-0567). The gapN expression was induced in *Corynebacterium glutamicum* by utilizing the characteristic of growing in a minimal medium by gapN expression. The CA01-0565, CA 01-0566, CA 02-0567 strains were deposited in KCCM (Korean Culture Center of Microorganisms, located at 361-221 Yurim Bild., Hongje 1-dong, Seodaemun-gu, Seoul, Korea) on July 2, 2009 with Accession NOS. KCCM 11013P, KCCM 11014P, and KCCM 11015P, respectively.

### Example 10. Examination of gapN activity in lysine-producing strain

Growth in a minimal medium using glucose as a carbon source was examined. As a result, the gapA-disrupted strain did not grow and the gapN-expressing strain recovered to grow.

**[Table 6] Growth**

| Strain | Growth | No growth |
|---|---|---|
| KFCC10881 | Growth | |
| CA01-0096 | | No growth |
| CA01-0565 | Growth | |
| CA01-0566 | Growth | |
| CA01-0567 | Growth | |

### Example 11. Examination of gapN activity in lysine-producing strain

The gapN expression levels were measured to confirm whether the gapN gene from the gapN expression vector was expressed in a cell so as to exhibit its activity. The CA01-0565, CA01-0566, and CA01-0567 strains were prepared by introducing each of the pECCG122-Pcj7-gapN1,2,3 vectors into the gapA-disrupted strain, the gapA-disrupted CA01-0096 strain, and the gapA-existing KFCC-10881 strain were cultured in seed media for one day, and then diluted at O.D 600=0.2, and 25 ml of each cell was recovered at O.D 600=10. The gapN activity was determined by a method described in A. Soukri et al., Protein Expression and Purification; 25; (2002) 519-529. As a result, it was found that gapN1 has an at 30°C for 20 hour with shaking (220 rpm). Thereafter, 1 ml of each of the seed cultures was inoculated into a 250 ml-corner baffle flask containing 25 ml of the production medium and cultured at 35°C for 96 hours with shaking (200 rpm). After the completion of culture, HPLC analysis was performed to determine the amounts of the L-lysine produced by the strains. The concentrations of L-lysine in the cultures of *Corynebacterium glutamicum* KFCC-10881, CA01-0096, CA01-0565, CA01-0566, and CA01-0567 are summarized in the following Table 8. As a result, the gapA-disrupted strain did not produce lysine. It was found that lysine was produced by the gapN gene introduced into the gapA-disrupted strain, and the production amounts were also increased by 16~17%, compared to that of the mother strain KFCC-10881 (Table 8).

**[Table 8] Flask culture result**

| Strain name | Lysine (g/l) |
|---|---|
| KFCC-10881 | 42 |
| CA01-0096 | 0 |
| CA01-0565 | 50 |
| CA01-0566 | 48.8 |
| CA01-0567 | 46 |

### Effect

According to the present disclosure, *Corynebacterium* strain having an L-lysine productivity and a gapN activity is prepared by introduction of *Streptococcus* and *Bacillus-*derived gapN-encoding genes, and thus the reducing power is increased to supply energy required for lysine production, leading to enhancement in the L-lysine production. increased to supply energy required for lysine production, leading to enhancement in the L-lysine production.
## Claims

1. A *Corynebacterium* sp. strain having an activity of NADP-dependent glyceraldehyde-3-phosphate dehydrogenase and a productivity of L-lysine, in which a gene encoding endogenous glyceraldehyde-3-phosphate dehydrogenase (gapA) is inactivated, and a gene encoding exogenous NADP-dependent glyceraldehyde-3-phosphate dehydrogenase (gapN) is introduced.

2. The Corynebacterium sp. strain according to claim 1, wherein the gene encoding exogenous NADP-dependent glyceraldehyde-3-phosphate dehydrogenase is a gene encoding NADP-dependent glyceraldehyde-3-phosphate dehydrogenase of *Streptococcus mutans,* (ATCC 25175), *Streptococcus agalactie* (ATCC BAA-611), or *Bacillus cereus* (ATCC 14579).

3. The Corynebacterium sp. strain according to claim 2, wherein the gene of Streptococcus mutans, the gene of Streptococcus agalactie, and the gene of Bacillus cereus have the nucleotide sequences of SEQ ID NOS. 11, 12, and 13, respectively.

4. The Corynebacterium sp. strain according to claim 1, wherein the gene encoding endogenous glyceraldehyde-3-phosphate dehydrogenase has the nucleotide sequence of SEQ ID NO. 14.

5. The Corynebacterium sp. strain according to claim 1, wherein the Corynebacterium sp. strain is *Corynebacterium glutamicum* CA01-0565 (Accession No. KCCM 11013P), Corynebacterium glutamicum CA01-0566 (Accession No. KCCM 11014P), or Corynebacterium glutamicum CA01-0567 (Accession No. KCCM 11015P).

6. The Corynebacterium sp. strain according to claim 1, wherein the improved productivity of L-lysine is obtained from the reducing power increased by activation of the NADP-dependent glyceraldehyde-3-phosphate dehydrogenase.

7. A method for producing L-lysine, comprising the steps of culturing the Corynebacterium sp. Strain according to any one of claims 1 to 6; and recovering lysine from the cultured cells or the culture broth.

## Patentansprüche

1. Corynebacterium-sp.-Stamm mit Aktivität von NADP-abhängiger Glyceraldehyd-3-phsophatdehydrogenase und Produktivität von L-Lysin, wobei ein Gen, das für endogene Glyceraldehyd-3-phosphatdehydrogenase (gapA) kodiert, inaktiviert ist und ein Gen, das für exogene NADP-abhängige Glyceraldehyd-3-phosphatdehydrogenase (gapN) kodiert, eingeführt ist.

2. Corynebacterium-sp.-Stamm nach Anspruch 1, wobei das für exogene NADP-abhängige Glyceraldehyd-3-phosphatdehydrogenase kodierende Gen ein Gen ist, das für NADP-abhängige Glyceraldehyd-3-phosphatdehydrogenase von Streptococcus mutans (ATCC 25175), Streptococcus agalactie (ATCC BAA-611) oder Bacillus cereus (ATCC 14579) kodiert.

3. Corynebacterium-sp.-Stamm nach Anspruch 2, wobei das Gen von Streptococcus mutans, das Gen von Streptococcus agalactie und das Gen von Bacillus cereus die Nucleotidsequenzen der Seq.-ID Nr. 11, 12 bzw. 13 aufweisen.

4. Corynebacterium-sp.-Stamm nach Anspruch 1, wobei das für endogene Glyceraldehyd-3-phosphatdehydrogenase kodierende Gen die Nucleotidsequenz der Seq.-ID Nr. 14 aufweist.

5. Corynebacterium-sp.-Stamm nach Anspruch 1, wobei der Corynebacterium-sp.-Stamm Corynebacterium glutamicum CA01-0565 (Zugangsnr. KCCM 11013P), Corynebacterium glutamicum CA01-0566 (Zugangsnr. KCCM 11014P) oder Corynebacterium glutamicum CA01-0567 (Zugangsnr. KCCM 11015P) ist.

6. Corynebacterium-sp.-Stamm nach Anspruch 1, wobei die verbesserte Produktivität von L-Lysin aus der Reduktionskraft erhalten wird, die durch die Aktivierung der NADP-abhängigen Glyceraldehyd-3-phsophatdehydrogenase erhöht wird.

7. Verfahren zur Produktion von L-Lysin, welches die Schritte des Kultivierens des Corynebacterium-sp.-Stamms nach einem der Ansprüche 1 bis 6; und des Gewinnens von Lysin aus den kultivierten Zellen oder der Kulturbrühe umfasst.

## Revendications

1. Souche *Corynebacterium* sp. ayant une activité de glycéraldéhyde-3-phosphate déhydrogénase dépendant de NADP et une productivité de L-lysine, où un gène codant la glycéraldéhyde-3-phosphate déhydrogénase endogène (gapA) est inactivé, et un gène codant la glycéraldéhyde-3-phosphate déhydrogénase exogène dépendant de NADP (gapN) est introduit.

2. Souche Corynebacterium sp. selon la revendication 1, où le gène codant la glycéraldéhyde-3-phosphate déhydrogénase exogène dépendant de NADP est un gène codant la glycéraldéhyde-3-phosphate déhydrogénase dépendant de NADP de *Streptococcus mutans* (ATCC 25175), *Streptococcus agalactie* (ATCC BAA-611) ou *Bacillus cereus* (ATCC 14579).

3. Souche Corynebacterium sp. selon la revendication 2, où le gène du Streptococcus mutans, le gène de Streptococcus agalactie et le gène de Bacillus cereus ont les séquences de nucléotides de SEQ ID NOS 11, 12 et 13, respectivement.

4. Souche Corynebacterium sp. selon la revendication 1, où le gène codant la glycéraldéhyde-3-phosphate déhydrogénase endogène a la séquence de nucléotides de SEQ ID NO 14.

5. Souche Corynebacterium sp. selon la revendication 1, où la souche Corynebacterium sp. est Corynebacterium glutamicum CA01-0565 (N° Accès KCCM 11013P), Corynebacterium glutamicum CA01-0566 (N° d'Accès KCCM 11014P) ou Corynebacterium glutamicum CA01-0567 (N° d'Accès KCCM 11015P).

6. Souche Carynebacterium sp. selon la revendication 1, où la productivité améliorée de la L-lysine est obtenue de la puissance réductrice augmentée par l'activation de la glycéraldéhyde-3-phosphate déhydrogénase dépendant de NADP.

7. Méthode de production de L-lysine, comprenant les étapes consistant à cultiver la souche Corynebacterium sp. selon l'une quelconque des revendications 1 à 6 ; et récupérer la lysine des cellules cultivées ou du bouillon de culture.
